# EUROPÄISCHE PATENTANMELDUNG

(11) **EP 3 249 561 A1**
(43) Veröffentlichungstag der Anmeldung: **29.11.2017**
(21) Anmeldenummer: 16178135.6
(22) Anmeldetag: 06.07.2016
(51) Int. Cl.: G06F 19/00, G06F 19/10, G06Q 50/22

(54) **VERFAHREN UND SYSTEM ZUR DOKUMENTATION EINES DIAGNOSTISCHEN TESTS**

(30) Priorität: 25.05.2016 EP 16171334
(71) Anmelder: Siemens Healthcare GmbH, 91052 Erlangen (DE)
(72) Erfinder: Die Erfindernennung liegt noch nicht vor

(57) **Zusammenfassung**

Die Erfindung beruht auf der Idee erste Werte für patientenspezifische Parameter basierend auf einem maschinenlesbaren Protokoll zu erfassen. Weiterhin schlagen die Erfinder vor Anforderungen an den diagnostischen Test basierend auf den ersten Werten zu bestimmen sowie zweiten Werten für Ergebnisparameter basierend auf dem diagnostischen Test gemäß den Anforderungen zu erfassen. Dann wird ein patientenspezifischer Bericht, umfassend die Anforderungen an den diagnostischen Test sowie die zweiten Werte für die Ergebnisparameter, automatisch erzeugt. Die Erfindung ermöglicht damit die Arbeitsschritte eines diagnostischen Tests zu standardisieren zu automatisieren.

## Beschreibung

Genetische Tests erlauben es die genetische Ursachen für Erkrankungen sowie die Wahrscheinlichkeiten für den Ausbrauch von Erkrankungen mit hoher Präzision zu bestimmen. Dadurch ermöglichen genetische Tests Ärzten und Patienten Maßnahmen zur Vorsorge oder Behandlung individuelle auf das genetische Profil des Patienten abzustimmen. Dabei existieren zum einen spezifische Tests für bestimmte genetische Mutationen. Ein solcher spezifischer Test beantwortet eine spezifische diagnostische Fragestellung und liefert keine darüber hinaus gehenden Informationen. Ein prominentes Beispiel dafür ist der Test zur Diagnose von Mutationen in den Genen BRCA1 oder BRCA2. Weiterhin haben sich in den vergangenen Jahren Hochdurchsatzverfahren zur Gensequenzierung durchgesetzt, welche die schnelle und kostengünstige Sequenzierung ganzer Genome erlaubt. Eine solche Genomsequenzierung erlaubt es auch Anomalien im ganzen Genom eines Patienten zu finden, nach denen ursprünglich nicht gesucht wurde. Diese Hochdurchsatzverfahren halten Einzug in den klinischen Alltag und sind nicht mehr nur spezialisierten Forschungseinrichtungen zugänglich. Daher stellt sich mit zunehmender Dringlichkeit die Frage der Qualitätskontrolle im Umgang mit sensiblen genetischen Daten im klinischen Umfeld.

Grundsätzlich sind Ärzte dazu verpflichtet Patienten vor Durchführung eines diagnostischen Tests über die Risiken sowie die Aussagekraft des Tests zu informieren. Bevor der Test durchgeführt wird, muss der Patienten diesem in Form einer informierte Einwilligung zustimmen. So wird in der Offenlegungsschrift US 20030204418 A1 diskutiert, wie die informierte Einwilligung des Patienten dokumentiert werden kann. Mit dem Aufkommen genetischer Tests werden jedoch neue und erhöhte Anforderungen an die Information von Patienten gestellt. Neben medizinischen Risiken sind vermehrt ethische Gesichtspunkte bei der Information von Patienten zu berücksichtigen. Beispielsweise stellt sich die Frage, ob und in welchem Umfang Patienten über zufällig gefundene Anomalien informiert werden soll. Dennoch existiert bis heute kein Standard um die verschiedenen Arbeitsschritte eines genetischen Tests von der Information des Patienten bis zur Kommunikation der Ergebnisse des Tests zu dokumentieren und in den klinischen Arbeitsablauf zu integrieren. Diese Frage gewinnt zunehmend an Dringlichkeit, da im klinischen Alltag mehrere Abteilungen am Durchführen und Analysieren genetischer Tests involviert sind. Ohne eine standardisierte sowie automatisierte Dokumentation ist die Gefahr groß, dass es zu einer fehlerhaften Interpretation der vorherigen Arbeitsergebnisse an den jeweiligen Schnittstellen zwischen den Abteilungen kommt.

Vor diesem Hintergrund ist es Aufgabe der vorliegenden Erfindung die Dokumentation der Arbeitsschritte eines diagnostischen Tests, insbesondere eines genetischen Tests, zu standardisieren und zu automatisieren. Dies wird geleistet durch ein Verfahren nach Anspruch 1, durch ein System nach Anspruch 11, durch ein Computerprogrammprodukt nach Anspruch 14 sowie durch ein maschinenlesbares Medium nach Anspruch 15.

Nachstehend wird die erfindungsgemäße Lösung der Aufgabe in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung beruht auf der Idee erste Werte für patientenspezifische Parameter basierend auf einem maschinenlesbaren Protokoll zu erfassen. Weiterhin schlagen die Erfinder vor Anforderungen an den diagnostischen Test basierend auf den ersten Werten zu bestimmen sowie zweiten Werten für Ergebnisparameter basierend auf dem diagnostischen Test gemäß den Anforderungen zu erfassen. Dann wird ein patientenspezifischer Bericht, umfassend die Anforderungen an den diagnostischen Test sowie die zweiten Werte für die Ergebnisparameter, automatisch erzeugt. Die Erfindung ermöglicht damit die Arbeitsschritte eines diagnostischen Tests zu standardisieren zu automatisieren.

Gemäß einem weiteren Aspekt der Erfindung den Schritt des zweiten Bestimmens von Behandlungsoption basierend auf den Ergebnisparametern umfassen. Damit werden auch die Behandlungsoptionen standardisiert dokumentiert.

Gemäß einem weiteren Aspekt der Erfindung werden die ersten Werte, die Anforderungen, die zweiten Werte und ggf. die Behandlungsoptionen sowie der Bericht abrufbar gespeichert, insbesondere verschlüsselt. Dadurch kann das Verfahren sowie der Bericht besser kontrolliert werden. Weiterhin erhöht ein Verschlüsseln beim Abspeichern der einzelnen Datensätze die Datensicherheit.

Gemäß einem weiteren Aspekt der Erfindung werden die Anforderungen basierend auf einem hierarchischen Aufgabennetzwerk bestimmt, wobei das hierarchische Aufgabennetzwerk dazu ausgebildet die Anforderungen basierend auf den ersten Werten zu bestimmen. Ein solches hierarchisches Aufgabennetzwerk strukturiert und automatisiert die Bestimmung der Anforderungen. Dies ist insbesondere dann hilfreich, wenn sich kein direkter funktionaler Zusammenhang zwischen einzelnen, ersten Werten und den Anforderungen herstellen lässt.

Gemäß einem weiteren Aspekt der Erfindung werden die zweiten Werte in Abhängigkeit der Anforderungen an den diagnostischen Test erfasst. Das Ergebnis eines diagnostischen Tests kann eine Vielzahl von Informationen umfassen, und das Erfassen von zweiten Werten in Abhängigkeit der Anforderungen stellt sicher, dass die für den jeweiligen Patienten relevanten Werte erfasst werden.

Gemäß einem weiteren Aspekt der Erfindung werden die zweiten Werte aus einer Vielzahl dritter Werte für die Ergebnisparameter ausgewählt, wobei die dritten Werte auf dem diagnostischen Test beruhen. Das selektive Auswählen stellt weiterhin sicher, dass nur die für den jeweiligen Patienten relevanten Werte erfasst werden.

Gemäß einem weiteren Aspekt der Erfindung werden die ausgewählten dritten Werte gelöscht oder anonymisiert gespeichert. Dadurch wird die Datensicherheit für den jeweiligen Patienten erhöht.

Gemäß einem weiteren Aspekt der Erfindung handelt es sich bei dem diagnostischen Test um einen genetischen Test. Die Standardisierung und Automatisierung eines genetischen Tests ist von besonderer Bedeutung, da die Interpretation der Ergebnisse in Form der zweiten Werte bei genetischen Tests besonders aufwändig ist.

Gemäß einem weiteren Aspekt der Erfindung bestimmen die Anforderungen, welche Genabschnitte im Rahmen des diagnostischen Tests sequenziert und analysiert werden sollen, insbesondere ob das komplette Exom oder das komplette Genom des Patienten sequenziert und analysiert werden soll. Denn diese Anforderungen sind besonders relevant für die Interpretation der Ergebnisse in Form der zweiten Werte.

Gemäß einem weiteren Aspekt der Erfindung umfassen die zweiten Werte die statistische Signifikanz des genetischen Tests und/oder die Relevanz des genetischen Tests für den Patienten. Da die Interpretation der Ergebnisse in Form der zweiten Werte bei genetischen Tests besonders aufwändig ist, erhöht die standardisierte und automatisierte Dokumentation die Zuverlässigkeit des gesamten klinischen Arbeitsablaufs um den genetischen Test herum erheblich.

Gemäß einem weiteren Aspekt der Erfindung wird der diagnostische Test durchgeführt basiert auf den bestimmten Anforderungen sowie einer Gewebsprobe des Patienten, insbesondere mittels eines Hochdurchsatzverfahrens.

Die Erfindung betrifft weiterhin ein System zur Dokumentation eines diagnostischen Tests für einen Patienten umfassend eine erste Schnittstelle zum Erfassen der ersten Werte sowie eine Recheneinheit mit einem Prozessor und eine zweite Schnittstelle zum zweiten Erfassen der zweiten Werte. Das System ist dazu ausgebildet das zuvor beschriebene Verfahren und seine Aspekte auszuführen, indem die Schnittstelle und die Recheneinheit dazu ausgebildet sind die entsprechenden Verfahrensschritte auszuführen.

Gemäß einem weiteren Aspekt der Erfindung umfasst das System weiterhin eine Testplattform zum Durchführen des diagnostischen Tests basierend auf den bestimmten Anforderungen sowie einer Gewebsprobe des Patienten.

Die Erfindung betrifft auch ein Computerprogrammprodukt mit einem Computerprogramm sowie ein maschinenlesbares Medium. Eine weitgehend softwaremäßige Realisierung hat den Vorteil, dass auch schon bisher verwendete Systeme oder Recheneinheiten auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie beispielsweise eine Dokumentation und/oder zusätzliche Komponenten auch Hardware-Komponenten, wie z.B. Hardware-Schlüssel (Dongles etc.) zur Nutzung der Software, umfassen.

### Definitionen:

Unter einem "diagnostischen Test" ist ein in-vitro Test zu verstehen, dessen Ergebnis zur medizinischen Diagnose verwendet werden kann. Für einen diagnostischen Test wird eine Gewebsprobe eines Patienten entnommen. Insbesondere kann es sich um eine flüssige Gewebsprobe, beispielsweise Blut oder Speichel, oder um eine feste Gewebsprobe, beispielsweise aus Niere, Leber oder Gehirn, handeln. Dann wird die Gewebsprobe mit biochemischen Methoden in-vitro analysiert. Mit einem diagnostischen Test kann beispielsweise ein Krankheitserreger wie das Humane Immundefizienz-Virus oder eine Anomalie wie ein Glukose-6-Phosphat-Mangel diagnostiziert werden. Insbesondere kann ein diagnostischer Test darauf basieren, dass bestimmte Moleküle detektiert oder charakterisiert werden. Ein solcher Test wird im Folgenden auch als molekulardiagnostischer Test bezeichnet. Bei diesen Molekülen kann es sich beispielhaft um DNA-Abschnitte oder RNA-Abschnitte, insbesondere um miRNAs, Inhibitoren oder Enzyme handeln.

Unter einem "genetischen Test" ist ein diagnostischer Test zu verstehen, bei dem Geneabschnitte des Patienten sequenziert und analysiert werden. Die Sequenzierung des Genabschnitts kann mit Hochdurchsatzverfahren durchgeführt werden, insbesondere durch das sogenannte "Sequencing-by-Synthesis". Die Sequenzierung kann sowohl das Auslesen der Reihenfolge von Basenpaaren als auch weitergehende Untersuchungen, beispielsweise von Methylierungen, betreffen. Je nach Art des genetischen Tests können unterschiedliche Genabschnitte sequenziert und analysiert werden. Beispielsweise können vorgegebene Gene wie BRCA1 oder BRCA2, größere genetische Bereiche, das ganze Exom oder sogar das ganze Genome des Patienten sequenziert und analysiert werden. Sogenannte Gen-Panels ermöglichen es bestimmte Gene oder Genabschnitte für die Sequenzierung und Analyse auszuwählen.

Unter einer "Analyse" kann die Detektion und/oder Charakterisierung eines Mikroorganismus oder Stoffes verstanden werden. Bei dem Mikroorganismus kann es sich insbesondere um Bakterien oder Viren handeln. Weiterhin kann es sich bei dem Stoff um ein Molekül wie zuvor beschrieben handeln. Die Detektion und/oder Charakterisierung kann mit bekannten biochemischen Methoden erfolgen. Beispielhaft seien als biochemische Methoden die Chromatographie, die Polymerase-Kettenreaktion, die Kristallographie oder Immunassays basierend auf Antikörper-Bindungen genannt. Die Detektion und/oder Charakterisierung kann weiterhin auf funktionellen Eigenschaften des Mikroorganismus oder des Stoffes beruhen.

Insbesondere kann unter einer "Analyse" die Detektion und/oder Charakterisierung eines Moleküls, insbesondere eines DNA-Abschnitts oder RNA-Abschnitts, verstanden werden. Eine solche Analyse beruht auf der Sequenz des DNA-Abschnitts oder des RNA-Abschnitts, also auf einer Folge von Basen oder Basenpaaren. Dabei können insbesondere eine Vielzahl von DNA-Abschnitten eines Patienten analysiert werden, wobei die DANN-Abschnitte zusammen das komplette Exom oder Genom des Patienten darstellen können. Dabei können die RNA- oder DNA-Abschnitte miteinander aliniert oder relativ zueinander ausgerichtet werden. Weiterhin können einzelne RNA- oder DNA-Abschnitte relativ zu einer Referenzstruktur ausgerichtet werden. Bei der Referenzstruktur kann es sich insbesondere um ein Referenzgenom oder Teile eines Referenzgenoms handeln. Auch können bereits alinierte oder relativ zueinander ausgerichtete RNA- oder DNA-Abschnitte relativ zu der Referenzstruktur ausgerichtet werden. Weiterhin können Abweichungen der ausgerichteten RNA- oder DNA-Moleküle relativ zu der Referenzstruktur detektiert und/oder charakterisiert werden, typischer Weise durch bioinformatische Methoden. Beispielsweise kann es sich bei solchen Abweichungen um einen Einzelnukleotid-Polymorphismus oder die Insertion eines RNA- oder DNA-Abschnitts handeln. Bekannte Methoden, auch als "variant caller" bekannt, sind beispielsweise SAMtools, glftools, GATK und Atlas2. Weiterhin können die Abweichungen mittels genomischer Referenzdatenbanken detektiert und/oder charakterisiert werden. Beispielhaft seien als Referenzdatenbanken SNPeffect, dbSNP, DGVa, COSMIC, dbVAR, SIFT, GERP+, Annovar und VAAST genannt. Weiterhin können die Abweichungen derart detektiert und/oder charakterisiert werden, dass eine den Abweichungen bestimmte Phänotypen zugeordnet werden.

Unter den "patientenspezifische Parameter" sind Parameter zu verstehen, welche den körperlichen Zustand des Patienten charakterisieren. Die Werte für die patientenspezifischen Parameter werden herkömmlicherweise durch eine Anamnese erfasst. Bei den patientenspezifischen Parametern handelt es sich beispielhaft um das Alter, den Blutdruck, das Gewicht, die Augenfarbe, Drogenkonsum, die Krankengeschichte des Patienten und/oder die Krankengeschichte der Verwandten ersten Grades. Insbesondere können die patientenspezifischen Parameter das Auftreten vererbbarer Krankheiten betreffen. Vererbbare Krankheiten beruhen auf einer Abweichung eines Genoms gegenüber einem gesunden Referenzgenom. Weiterhin können die patientenspezifischen Parameter eine Gewebsprobe des Patienten betreffen, insbesondere die Art, die Gewinnung, Lagerung und/oder die Verarbeitung der Gewebsprobe. Beispielhaft kann die Lagerung der Gewebsprobe eine Kryo-Lagerung oder eine Formalin-basierte lagerung betreffen. Beispielhaft kann die Verarbeitung der Gewebsprobe die Autolysezeit oder die Homogenität der Gewebsprobe betreffen.

Unter einem "maschinenlesbares Protokoll" ist ein Computerprogramm oder ein Computerprogrammabschnitt zu verstehen, bei dessen Ausführung festgelegte patientenspezifische Parameter erfasst werden. Da festgelegte patientenspezifische Parameter durch das Protokoll erfasst werden, wird der Schritt des Erfassens gegenüber einer herkömmlichen Anamnese standardisiert und automatisiert. Das Protokoll kann derart ausgebildet sein, dass erste Werte für eine erste Klasse patientenspezifischer Parameter sowie weiterer erster Werte für eine zweite Klasse patientenspezifischer Parameter erfasst werden, wobei die zweite Klasse abhängig ist von der ersten Klasse. Beispielsweise können die ersten Werte für die erste Klasse patientenspezifischer Parameter implizieren, dass mit erhöhter Wahrscheinlichkeit eine bestimmte vererbbare Krankheit vorliegt. Dann ist es sinnvoll weitere erste Werte für die zweite Klasse patientenspezifischer Parameter zu erfassen, welche ein besonders gutes Maß für die Wahrscheinlichkeit sind, dass die bestimmte vererbbare Krankheit vorliegt.

Das "Erfassen" erster Werte oder zweiter Werte umfasst, dass diese Werte wenigstens teilweise automatisch erfasst werden. Insbesondere können die Werte dabei in den internen Speicher einer Recheneinheit geladen und/oder abrufbar gespeichert werden. Die Werte können dabei über eine Schnittstelle übertragen werden. Insbesondere die ersten Werte können über eine graphische Benutzeroberfläche übertragen werden, wobei eine Bedienperson die ersten Werte in die graphische Benutzeroberfläche eingibt und/oder auswählt. Insbesondere die zweiten Werte können vollautomatisch erfasst werden, wobei beispielsweise eine Testplattform die zweiten Werte erzeugen und diese über eine Schnittstelle an eine Recheneinheit übertragen kann. Beispielhaft und ohne Beschränkung können die ersten Werte in einem im Format XML oder JSON erfasst werden.

Die "ersten Werte" sowie die "zweiten Werte" können sowohl als numerische Werte als auch in anderer maschinenlesbarer Form vorliegen. Beispielsweise können die ersten Werte in Textform über eine graphische Benutzeroberfläche ausgewählt werden. Die Werte können durch die Recheneinheit in numerische Werte umgewandelt werden. Damit können insbesondere mathematische Rechenoperationen wie Vergleiche auf die Werte angewendet werden.

Unter den "Anforderungen" an den diagnostischen Test sind Anforderungen an die Fähigkeiten und/oder Genauigkeit des diagnostischen Tests gemeint bestimmte Mikroorganismen oder Stoffe zu detektieren und/oder zu charakterisieren. Beispielsweise kann eine Anforderung die Fähigkeit und/oder Genauigkeit betreffen, mit der einer Abweichungen ausgerichteter RNA- oder DNA-Moleküle relativ zu einer Referenzstruktur detektiert und/oder charakterisiert werden kann. Eine Anforderung kann auch die Art des Assays für den diagnostischen Test betreffen, wobei es sich bei dem Assay beispielsweise um eine Analyse des kompletten Genoms des Patienten durch ein Hochdurchsatzverfahren, insbesondere "Sequencing-by-Synthesis" handeln kann. Weiterhin kann die Anforderung die Art der Abweichung betreffen, beispielsweise in Form eines Einzelnukleotid-Polymorphismus. Weiterhin kann eine Anforderung die Fähigkeit und/oder Genauigkeit betreffen, mit der eine bestimmte Abweichung, beispielsweise eine Mutation in BRCA1 oder BRCA2, detektiert und/oder charakterisiert werden kann.

Unter einem "Ergebnisparameter" ist ein Parameter zu verstehen, welcher wenigstens einen Teil des Ergebnisses des diagnostischen Tests darstellt. Ein Ergebnisparameter kann insbesondere die Anwesenheit eines Mikroorganismus oder eines Stoffes betreffen. Weiterhin kann der Ergebnisparameter quantitative Abweichungen ausgerichteter RNA- oder DNA-Moleküle relativ zu einer Referenzstruktur charakterisieren. Beispielsweise kann der Ergebnisparameter die Häufigkeit betreffen, mit der eine Mutation in bestimmten Genen detektiert worden ist sowie die Zuverlässigkeit, mit der eine Mutation in bestimmten Genen charakterisiert worden ist.

Unter "Behandlungsoptionen" sind eine oder mehrere Behandlungsmethoden des Patienten zu versehen. Insbesondere können sich die Behandlungsoptionen auf bestimmte Medikamente und deren Dosis beziehen. Die Behandlungsoptionen werden wenigstens abhängig von den zweiten Werten bestimmt. Sie können aber auch abhängig von den ersten Werten und/oder den Anforderungen bestimmt werden.

Unter einem "Bericht" ist eine Datei oder ein Dokument zu verstehen, welches die Anforderungen an den diagnostischen Test sowie die zweiten Werte für die Ergebnisparameter umfasst. Ein solcher Bericht weist eine vorgegebene Struktur auf. Beispielsweise kann der Bericht eine vorgegebenen logische Struktur aufweisen, gemäß derer die Anforderungen den ersten Werten und/oder den zweiten Werten zugeordnet sind. Weiterhin kann die Struktur des Berichts von dem maschinenlesbaren Protokoll abhängen. Umfasst das Verfahren zur Dokumentation eines diagnostischen Test die Auswahl eines maschinenlesbaren Protokolls aus einer Menge vorgegebenen maschinenlesbaren Protokolle, dann kann damit auch eine bestimmte Struktur für den Bericht ausgewählt werden. Die Struktur kann insbesondere die graphische Anordnung der Anforderungen sowie der zweiten Werte bei der Ausgabe des Berichts betreffen.

Die einzelnen Schritte des beschriebenen Verfahrens können automatisch oder vollautomatisch erfolgen. "Automatisch" bedeutet im Kontext der vorliegenden Anmeldung, dass der jeweilige Schritt mittels einer Recheneinheit und/oder eines Computerprogramms selbstständig abläuft, und für den jeweiligen Schritt im Wesentlichen keine Interaktion einer Bedienperson mit dem System zur Dokumentation eines diagnostischen Tests mit notwendig ist. Die Bedienperson muss höchstens berechneten Ergebnisse bestätigen oder Zwischenschritte ausführen. In weiteren Ausführungsformen der Erfindung mit "vollautomatisch" durchgeführten Schritten ist zur Durchführung dieser Schritte gar keine Interaktion einer Bedienperson notwendig. Unabhängig davon, ob die einzelnen Schritte "automatisch" oder "vollautomatisch" ausgeführt werden, kann das erfindungsgemäße Verfahren Bestandteil eines Arbeitsablaufes sein, der zusätzlich eine Interaktion von einer Bedienperson erfordert, insbesondere die Eingabe der ersten Werte durch eine graphische Benutzeroberfläche.

Ein "hierarchisches Aufgabennetzwerk" ist im Englischen auch als "hierarchical task network" bekannt. Beispielhaft und ohne Beschränkung kann das hierarchische Aufgabennetzwerk durch eine der folgenden Softwares gegebene sein: SIPE-2, O-Plan, UMCP, SHOP2, HTNPlan-P. Ein hierarchisches Aufgabennetzwerk beschreibt die Abhängigkeit zwischen patientenspezifischen Parametern, Anforderungen sowie den Ergebnisparametern. Ein Aufgabennetzwerk weist Aufgaben auf, welche durch Randbedingungen eingeschränkt werden. Diese Randbedingungen können durch patientenspezifische Parameter gegeben sein. Die Aufgabe kann darin bestehen die Anforderungen zu bestimmen sowie den Bericht zu erzeugen. Ein hierarchisches Aufgabennetzwerk kann unterschiedliche Typen von Aufgaben aufweisen, insbesondere primitive Aufgaben, zusammengesetzte Aufgaben und Zielaufgaben. Ein hierarchisches Aufgabennetzwerk ist insofern hierarchisch, als dass ein bestimmter Aufgabentyp ausgeführt werden muss, bevor ein anderer Aufgabentyp ausgeführt werden kann. Beispielsweise können zusammengesetzte Aufgaben aus primitiven Aufgaben zusammengesetzt sein. Dann müssen die primitiven Aufgaben ausgeführt werden, damit die zusammengesetzte Aufgabe ausgeführt werden kann. Beispielsweise kann das Erfassen der ersten Werte in Form mehrerer primitiver Aufgaben ausgebildet sein, und das erste Bestimmen der Anforderungen kann als zusammengesetzte Aufgabe ausgebildet sein. Weiterhin können das automatische Erzeugen eines Berichts und/oder das Bestimmen von Behandlungsoptionen als Zielaufgabe ausgebildet sein. Zielaufgaben werden in Form von Bedingungen spezifiziert. Solche Bedingungen können durch die zweiten Werte gegeben sein. Beispielsweise kann die Bedingung lauten, dass eine bestimmte Mutation in einem bestimmten Gen des Patienten vorliegt. Dann das Bestimmen der Behandlungsoptionen als Zielaufgabe ausgebildet sein.

Bei einer "Schnittstelle" handelt es sich um allgemein bekannte Hard- oder Software-Schnittstellen, z.B. um die Hardware-Schnittstellen PCI-Bus, USB oder Firewire, oder um eine Software-Schnittstelle wie Bluetooth. Weiterhin können die ersten Werte erfasst werden, indem sie durch eine Eingabeeinheit eingegeben werden, welche eine Schnittstelle aufweist oder mit einer solchen verbunden ist. Auch kann der Bericht erzeugt werden, indem er durch eine Ausgabeeinheit ausgegeben wird, welche eine Schnittstelle aufweist oder mit einer solchen verbunden ist. Die Eingabeeinheit kann als berührungsempfindlicher Bildschirm, Tastatur, Maus, Mikrofon zur Spracheingabe oder anderweitig ausgebildet sein. Die Ausgabeeinheit kann als Bildschirm, Drucker, Lautsprecher oder anderweitig ausgebildet sein.

Eine "Recheneinheit" kann sowohl Software- als auch Hardwareelement aufweisen und umfasst mindestens einen Prozessor.

Bei dem Prozessor kann es sich insbesondere um einen Mikroprozessor handeln. Die Recheneinheit kann über einen Speicher verfügen, insbesondere um Werte, Anforderungen sowie Berichte abrufbar zu speichern. Weiterhin kann die Recheneinheit als Teil eines Netzwerkes, insbesondere als Client oder Server, ausgebildet sein. Auch kann das System zur Dokumentation eines diagnostischen Tests sowohl einen Client, insbesondere als Eingabeeinheit, als auch einen Server umfassen. Die Recheneinheit kann mit einem maschinenlesbaren Medium zusammenwirken, insbesondere um durch ein Computerprogramm mit Programmcode ein erfindungsgemäßes Verfahren durchzuführen. Weiterhin kann das Computerprogramm auf dem maschinenlesbaren Medium abrufbar gespeichert sein. Insbesondere kann es sich bei dem maschinenlesbaren Medium um eine CD, DVD, Blu-Ray Disc, einen Memory-Stick oder eine Festplatte handeln. Das Computerprogramm kann insbesondere als ausführbare Datei ausgebildet sein.

### Beschreibung der Figuren

- Fig. 1: zeigt eine Übersichtsdarstellung eines Ablaufs gemäß einer bevorzugten Ausführungsform der Erfindung.
- Fig. 2: zeigt ein Ablaufdiagramm für einen integrierten Workflow gemäß einer weiteren bevorzugten Ausführungsform der Erfindung.
- Fig. 3: stellt ein Ablaufdiagramm für ein Verfahren zur Dokumentation eines diagnostischen Tests gemäß einer weiteren bevorzugten Ausführungsform der Erfindung dar und
- Fig. 4: zeigt ein Blockdiagramm für ein System zur Dokumentation in einer vorteilhaften Ausführungsform der Erfindung.

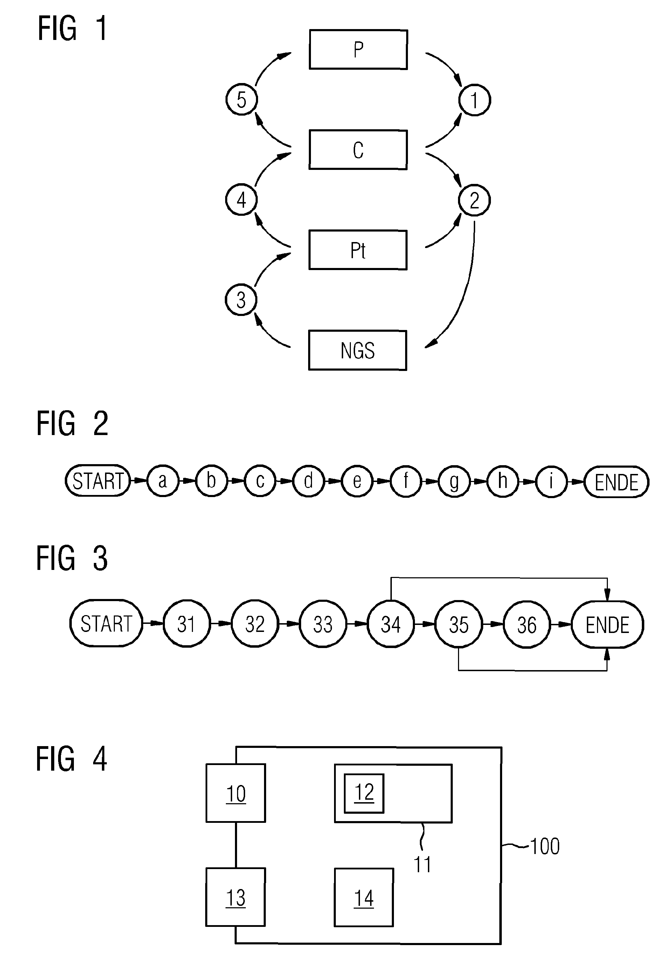

Unter Bezugnahme auf Fig. 1 wird das Verfahren in einer beispielhaften Umsetzung erläutert. Wie mit den Ziffern 1 bis 5 in Fig. 1 repräsentiert, werden 5 Hauptschritte durchgeführt:
1.) In einem ersten Schritt wird ein Aufklärungsgespräch (Counselling) ausgeführt und die Patienteneinwilligung wird eingeholt (Informed consent). Dabei interagieren der Patient P und der Arzt C oder ein Mitarbeiter eines Krankenhauses. Dies kann über einen Computer erfolgen.
2.) In Schritt 2 erfolgt die Auswahl der Gewebeprobe und eine Spezifikation der technischen NGS Parameter. Diese Datensätze können in elektronischer Form zwischen dem Arzt C und dem Pathologen Pt ausgetauscht werden.
3.) In Schritt 3 erfolgt die NGS Analyse seitens des NGS-Labors NGS. In Schritt 3 kann nach Ausführung des genetischen Tests oder einer anderen Analyse ein Analysebericht bereitgestellt werden. Weiterhin kann nach Empfang des Berichts eine Qualitätskontrolle ausgeführt werden, die z.B. mit einer Versendung eines Qualitätsberichtes an das Labor NGS endet.
4.) In Schritt 4 kann die molekular biologische Diagnose von dem Pathologen Pt an den Arzt C bereitgestellt werden.
5.) In Schritt 5 werden die abschließenden Schritte ausgeführt, wie das Erstellen eines Berichts, das Aufklärungs- oder Beratungsgespräch zwischen Arzt C und Patient P und die Mitteilung der klinischen Entscheidung auf Basis des durchgeführten Tests/der Analyse.

Für den Schritt 1 und die Durchführung des Arztgespräches können u.a. folgende Faktoren zu berücksichtigen sein:
1. klinische Parameter
2. Anamnese, Familienkrankengeschichte
3. Liste von Diagnostischen Optionen
4. Listen der Probenparameter
5. Liste von therapeutischen Optionen

Vor der Zustimmungserklärung (informed consent) erfolgt ein Aufklärungsgespräch, in dem der Patient P angemessen auf die Eigenschaften des Tests, die Tragweite der Ergebnisse, die Testbeschränkungen, mögliche Nebenwirkungen, sowie Relevanz der Testergebnisse für den Patienten P und seine Angehörigen hingewiesen wird. Diese Informationen werden erfindungsgemäß automatisch in einer strukturierten Form dokumentiert.

Das Ergebnis ist eine Zustimmungserklärung mit:
1. Art der (molekularen) Analyse
2. Beschreibung möglicher Überschussinformation
3. Optionen für den Umgang mit Überschussinformation (Löschen, Verwendung für Forschung, Bericht an den Patienten)
4. Dokumentation des Beratungsprozesses.

### Beispiel 1:

1. Klinische Parameter
   - Histologisch bestätigtes kolorektales Karzinom
   - Tumorlokalisation
   - Tumorstadium
   - Erkrankungsalter: <50
   - Polypen: keine; >10; >10 und <100; >100
2. Anamnese, Familienkrankengeschichte
   - Krebs: JA
      - Kolorektal
      - Endometrium
      - Magen
      - Gallenwege Urothel (Nierenbecken und Harnlei-ter) DünndarmPankreas
      - Zentrales Nervensystem
      - Ovar
   - Tumoren der Talgdrüsen Aufgetreten bei:
      i. Verwandte ersten Grades
      ii. Verwandte anderen Grades mit mehr als 2 Krebsfällen
      iii. Minimum 2 Generationen
      iv. Ein Krebsfall jünger als 50Jahre
      v. Keine familiäre Polypose
3. Liste von diagnostischen Optionen
   - Mikrosatteliten Instabilität
   - Immunhistochemische Untersuchung von MLH1
   - Mutationsanalyse von
      - MLH1
      - MSH6
      - PMS2
      - EPCAM
      - APC
      - MUTYH
      - KRAS
      - BRAF
      mittels
      - Protein truncation test (PTT)
      - Denaturing High Performance Liquid Chromatog-raphy (DHPLC)
      - Single strand conformation polymorphism (SSCP)
      - Conformation sensitive gel electrophoresis (CSGE)
      - Polymerasekettenreaktion
      - Sequenzierung
         i. Sanger
         ii. Pyrosequenzierung
         iii. "Next generation sequencing" (NGS)
4. Liste der Probenparameter
   a. Gefierkonserviertes Gewebe
   b. Formalin-fixiertes und Paraffin-eingebettetes (FFPE) Gewebe
      i. Tumorgewebe
         1. Erhaltungszustand
         2. Tumorzellanteil
         3. Nekrosen
      ii. Nicht-neoplastisches Gewebe
   c. Blutproben
      i. Frisches Blut
      ii. Isolierte genomische DNA
      iii. Plasma
      iv. Frei-zirkulierende DNA
5. Liste von therapeutischen Optionen
   a. Operation
   b. Medikamente
      i. 5-Flourouracil
      ii. Folinsäure
      iii. Levamisol
      iv. Capecitabin
      v. Oxaliplatin
      vi. Irinotecan
      vii. Cetuximab
      viii.Panitumumab
      ix. Bevacizumab
   c. Radiotherapie

### Beispiel 2:

1. Klinische Parameter
   a. Erkrankungsalter: >50
   b. Metastasiertes Karzinom des Kolons
   c. Polypen: keine
2. Anamnese, Familienkrankengeschichte
   a. Krebs: NEIN
3. Liste von Diagnostische Optionen
   a. KRAS Mutationsanalyse mittels NGS
4. Liste von therapeutischen Optionen
   a. Therapie mit Cetuximab

Bei Schritt 2 (Auswahl der Gewebeprobe und Spezifikation der technischen NGS Parameter) ist der Ausgangpunkt, das Ergebnis aus Schritt 1 (z.B. Vorbereitete Gewebeprobe, Technische Parameter für die molekulare (NGS) Analyse, z. B. Tumormaterial eines FFPE Blocks mit einem Tumorzellanteil von 70%; gute Gewebserhaltung; keine Nekrosen)

Bei Schritt 3 (NGS Analyse) ist der Ausgangpunkt, das Ergebnis (auch mehrere Ergebnisse sind möglich) aus Schritt 2. Das Ergebnis kann z. B sein:
Liste von Gen varianten (Mutation) mit zugehöriger Qualitätsinformation. Abhängig von der Zustimmungserklärung wir Überschussinformation in Schritt 3 gelöscht, anonymisiert für die Forschung gespeichert, oder an Schritt 4 weitergegeben.

### Beispiel NGSAnalyse (AMLv1 Panel):

### Diagnose Relevant:

- KRAS

### Überschussinformation:

- ASXL1, CEBPA, DNMT3A, EZH2, FLT3, HRAS, IDH1, IDH2, JAK2, KIT, MLL, MPL, NPM1, NRAS, PHF6, PTEN, RUNX1, TET2, TP53, WT1

Bei Schritt 4 (Pathologische Diagnose) ist der Ausgangpunkt, das Ergebnis Schritt 3.

### Beispiel Molekularpathologische Beschreibung:

Untersuchung: Nachweis von Mutationen im KRAS Gen Ergebnis: Bei der Untersuchung von Paraffinmaterial (Tumorgewebe mit Tumorzellanteil von 70%) isolierter DNA mittels NGS (AMLv1 Panel) ist keine Mutation des KRAS Gens Molekularpathologische Diagnose: Im vorliegenden Untersuchungsmaterial (Tumorgewebe, Paraffinblock) ist eine Mutation des KRAS Gens nicht nachweisbar.

Kommentar: Etwa 30%-50% der Kolonkarzinome weisen eine Mutation im KRAS Onkogen auf. Die meisten Mutationen treten dabei in codons 12 und 13 auf. Diese wurden als negativer voraussage Parameter für die Wirkung von anti-epidermal growth factor antibodies (EGFR) validiert. 40%-60% der Patienten mit wildtype KRAS reagieren auf EGFR Behandlung nicht. In diesen Fällen kann eine Mutation im BRAF gen, das bei 5%-10% der Tumore mutiert ist, die Reaktion auf die Behandlung beeinflussen. (TAN, CONG et al.: "KRAS mutation testing in metastatic colorectal cancer", in: World J Gastroenterology, 2012, Vol. 18, Iss. 37, pp. 5171-5180, DOI:10.3748/wjg.v18.i37.5171 and WILSON, PM et al.: "Molecular markers in the treatment of metastatic colorectal cancer", in: Cancer J., 2010, Vol. 16, No. 3, pp. 262-272).

In Schritt 5 (Klinische Entscheidung und Patientengespräch) dient als Ausgangpunkt wieder das Ergebnis aus Schritt 4.

### Beispiel:

KRAS nicht mutiert daher Behandlung mit EGFR Inhbitor Cetuximab. Daran anschließend erfolgt eine Dokumentation des Patientengesprächs.

Figur 2 zeigt ein Ablaufdiagramm zur Ausführung eines integrierten Workflows von einer medizinischen Fragestellung bis zur Erstellung eines klinischen Berichts gemäß einer bevorzugten Ausführungsform der Erfindung. Nach dem Start erfolgt in Schritt a das Erfassen der medizinischen Fragestellung. In Schritt b wir das Beratungsgespräch durchgeführt. Nachfolgend kann in Schritt c eine Patienteneinwilligung bzw. Einverständniserklärung vom Patienten (informed consent) abgegeben oder vom Arzt eingeholt werden. In Schritt d kann auf Basis dieser Daten ein Auftrag an das Gen-Labor erstellt und ausgegeben werden. In Schritt e kann ein Management der genetischen Proben ausgeführt werden, um diese dann - ggf. nachfolgend - in Schritt f eine Voranalyse (pre-analytics) zu unterziehen. Anschließend kann in Schritt g das Ausführen des genetischen Testverfahrens ausgeführt werden. Das technische Ergebnis des Testverfahrens wird in Schritt h bereitgestellt und kann im letzten Schritt i in einen klinischen Bericht einfließen.

Fig. 3 zeigt ein weiteres Ablaufdiagramm zur Ausführung eines Verfahrens zur Dokumentation eines diagnostischen Tests für einen Patienten. In Schritt 31 werden erste Werte für patientenspezifische Parameter erfasst. Dies kann auf Basis eines maschinenlesbaren Protokolls erfolgen. In Schritt 32 werden Anforderungen an den diagnostischen Test, basierend auf den erfassten ersten Werten bestimmt. In Schritt 33 werden zweite Werte für Ergebnisparameter, basierend auf dem diagnostischen Test gemäß den bestimmten Anforderungen erfasst. In Schritt 34 wird automatisch ein patientenspezifischer Bericht, umfassend die Anforderungen an den diagnostischen Test sowie die (zweiten) Werte für die Ergebnisparameter erzeugt. Danach kann das Verfahren enden.

In einer bevorzugten Ausführungsform der Erfindung kann in Schritt 35 ein zweites Bestimmen von Behandlungsoptionen, basierend auf den Ergebnisparametern, ausgeführt werden.

In einer bevorzugten Ausführungsform der Erfindung kann in Schritt 36 ein Durchführen des diagnostischen Tests, basierend auf den bestimmten Anforderungen sowie einer Gewebeprobe des Patienten, insbesondere mittels eines Hochdurchsatzverfahrens, durchgeführt werden.

Die Schritte 35 und 36 sind jedoch lediglich optional.

Fig. 4 zeigt ein Blockdiagramm für ein System 100 zur Dokumentation eines diagnostischen Tests für einen Patienten, wobei das System 100 mehrere elektronische oder informationstechnologische Module 10, 11, 11, 13, 14 umfasst.

Eine erste Schnittstelle 10 dient zum Erfassen von ersten Werten für patientenspezifische Parameter, basierend auf einem maschinenlesbaren Protokoll.

Eine Recheneinheit 11 umfasst einen Prozessor 12, wobei der Prozessor 12 programmiert ist, um ein erstes Bestimmen von Anforderungen an den diagnostischen Test, basierend auf den ersten Werten auszuführen.

Eine zweite Schnittstelle 13 dient zum zweiten Erfassen von zweiten Werten für Ergebnisparameter, basierend auf dem diagnostischen Test gemäß den Anforderungen.

Der Prozessor 12 ist weiterhin programmiert, um automatisch einen patientenspezifischen Bericht zu erzeugen, umfassend die Anforderungen an den diagnostischen Test sowie die zweiten Werte für die Ergebnisparameter.

In einer bevorzugten Ausführungsform der Erfindung kann das System 100 des Weiteren eine Testplattform 14 zum Durchführen des diagnostischen Tests, basierend auf den bestimmten Anforderungen sowie einer Gewebeprobe des Patienten umfassen.

## Patentansprüche

1. Verfahren zur Dokumentation eines diagnostischen Tests für einen Patienten umfassend folgende Schritte:
- erstes Erfassen (31) von ersten Werten für patientenspezifische Parameter basierend auf einem maschinenlesbaren Protokoll,
- erstes Bestimmen (32) von Anforderungen an den diagnostischen Test basierend auf den ersten Werten,
- zweites Erfassen (33) von zweiten Werten für Ergebnisparameter basierend auf dem diagnostischen Test gemäß den Anforderungen,
- automatisches Erzeugen (34) eines patientenspezifischen Berichts, umfassend die Anforderungen an den diagnostischen Test sowie die zweiten Werte für die Ergebnisparameter.

2. Verfahren nach Anspruch 1, weiterhin umfassend:
- zweites Bestimmen von (35) von Behandlungsoptionen basierend auf den Ergebnisparametern.

3. Verfahren nach einem der vorhergehenden Ansprüche, wobei die Anforderungen basierend auf einem hierarchischen Aufgabennetzwerk bestimmt werden, wobei das hierarchische Aufgabennetzwerk dazu ausgebildet ist, die Anforderungen basierend auf den ersten Werten zu bestimmen.

4. Verfahren nach einem der vorhergehenden Ansprüche, wobei die zweiten Werte in Abhängigkeit der Anforderungen an den diagnostischen Test erfasst werden.

5. Verfahren nach Anspruch 4, wobei die zweiten Werte aus einer Vielzahl dritter Werte für die Ergebnisparameter ausgewählt werden, wobei die dritten Werte auf dem diagnostischen Test beruhen.

6. Verfahren nach Anspruch 5, wobei die nicht ausgewählten dritten Werte gelöscht oder anonymisiert gespeichert werden.

7. Verfahren nach einem der vorhergehenden Ansprüche, wobei es sich bei dem diagnostischen Test um einen genetischen Test handelt.

8. Verfahren nach Anspruch 7, wobei die Anforderungen bestimmen, welche Genabschnitte im Rahmen des diagnostischen Tests sequenziert und analysiert werden sollen, insbesondere ob das komplette Exom oder das komplette Genom des Patienten sequenziert und analysiert werden soll.

9. Verfahren nach Anspruch 7 oder 8, wobei die zweiten Werte die statistische Signifikanz des genetischen Tests und/oder die Relevanz des genetischen Tests für den Patienten umfassen.

10. Verfahren nach einem der vorherigen Ansprüche, weiterhin umfassend:
- Durchführen (36) des diagnostischen Tests basierend auf den bestimmten Anforderungen sowie einer Gewebsprobe des Patienten, insbesondere mittels eines Hochdurchsatzverfahrens.

11. System (100) zur Dokumentation eines diagnostischen Tests für einen Patienten umfassend folgende Module:
- erste Schnittstelle (10) zum Erfassen von ersten Werten für patientenspezifische Parameter basierend auf einem maschinenlesbaren Protokoll,
- Recheneinheit (11) mit einem Prozessor (12), wobei der Prozessor (12) programmiert zum ersten Bestimmen von Anforderungen an den diagnostischen Test basierend auf den ersten Werten,
- zweite Schnittstelle (13) zum zweiten Erfassen von zweiten Werten für Ergebnisparameter, basierend auf dem diagnostischen Test gemäß den Anforderungen,
wobei der Prozessor (12) weiterhin programmiert ist zum automatischen Erzeugen eines patientenspezifischen Berichts, umfassend die Anforderungen an den diagnostischen Test sowie die zweiten Werte für die Ergebnisparameter.

12. System nach Anspruch 11, wobei die Schnittstelle (10, 13) und die Recheneinheit (11) dazu ausgebildet sind ein Verfahren nach einem der Ansprüche 2 bis 10 auszuführen.

13. System nach Anspruch 11 oder 12, weiterhin umfassend eine Testplattform (14) zum Durchführen des diagnostischen Tests basierend auf den bestimmten Anforderungen sowie einer Gewebeprobe des Patienten.

14. Computerprogrammprodukt mit einem Computerprogramm, welches direkt in einen Speicher eines Systems zum zur automatischen Dokumentation eines diagnostischen Tests ladbar ist, mit Programmabschnitten, um alle Schritte des Verfahrens nach einem der Ansprüche 1 bis 10 auszuführen, wenn die Programmabschnitte von dem System zur automatischen Dokumentation eines diagnostischen Tests ausgeführt werden.

15. Maschinenlesbares Medium, auf welchem von einer Recheneinheit lesbare und ausführbare Programmabschnitte gespeichert sind, um alle Schritte des Verfahrens nach einem der Ansprüche 1 bis 10 auszuführen, wenn die Programmabschnitte von dem System zur automatischen Dokumentation eines diagnostischen Tests ausgeführt werden.
